# EUROPEAN PATENT SPECIFICATION

(11) **EP 4 041 339 B1**
(45) Date of publication and mention of the grant of the patent: **16.07.2025**
(21) Application number: 20789228.2
(22) Date of filing: 02.10.2020
(51) Int. Cl.: A61M 5/14, A61M 5/168, A61M 5/172, G06F 3/01, G16H 20/17

(54) **HAND OPERATED DEVICE FOR ENABLING A PATIENT CONTROL OF LEVEL OF SEDATION TO FIT AROUND A WRIST AND HAVING A SWITCH**
HANDBETÄTIGTE, UM EIN HANDGELENK PASSENDE VORRICHTUNG ZUM ERMÖGLICHEN DER STEUERUNG DES SEDIERUNGSGRADES EINES PATIENTEN MIT EINEM SCHALTER
DISPOSITIF À COMMANDE MANUELLE PERMETTANT À UN PATIENT DE CONTRÔLER LE NIVEAU DE SÉDATION, À AJUSTER AUTOUR D'UN POIGNET ET AYANT UN COMMUTATEUR

(30) Priority: 02.10.2019 GB 201914214
(43) Date of publication of application: 17.08.2022
(73) Proprietor: Nottingham University Hospitals NHS Trust, Nottingham, Nottinghamshire NG7 2UH (GB)
(72) Inventor: BEDFORTH, Nigel, Nottingham Nottinghamshire NG7 2UH (GB); HEWSON, David, Nottingham Nottinghamshire NG7 2UH (GB); SPRINKS, James, Nottingham Nottinghamshire NG1 4FQ (GB); WORCESTER, Frank, Nottingham Nottinghamshire NG1 4FQ (GB); BREEDON, Philip, Nottingham Nottinghamshire NG1 4FQ (GB)
(74) Representative: Barker Brettell LLP
(86) International application number: PCT/GB2020/052422
(87) International publication number: WO 2021/064415

(56) References cited:
- EP-A1- 0 523 456
- EP-A1- 1 338 295
- WO-A1-03/057296
- WO-A1-2013/136176
- WO-A1-2016/014311
- US-A1- 2008 220 092

## Description

### Field of the Invention

The invention relates to patient-maintained sedation and in particular to a novel hand-operated device for enabling patients to control their own level of sedation.

### Background

In the UK in 2013 approximately 3.6 million operations were carried out. Of these, 2.8 million were performed with the patient unconscious (under general anaesthesia), and 0.8 million were carried out with the patient awake. Operations performed with the patient awake are associated with an increased risk of patient anxiety and pain, whereas operations under general anaesthesia carry risks associated with the anaesthetic. For many operations, deep sedation and anaesthesia is not necessary, provided the problems of anxiety and pain can be managed. This can be achieved by careful control of a level of sedation of the patient, in combination with local anaesthesia where appropriate. The ideal level of sedation during a procedure is highly patient dependent. Pharmacokinetic factors (how the body deals with the sedative agent) include age, weight and less easily determined factors such as metabolic rate and proportion of body fat. Most importantly though, patients differ widely in their anxiety levels and their need for sedation during the procedure. Sedatives are usually administered by an anaesthetist. The patient is therefore not in control of how much sedation they receive. In this situation the patient may receive too little sedation; resulting in increased anxiety and a poor subjective experience. Conversely the patient may receive too much sedation; with possible complications such as low blood pressure, respiratory depression, airway obstruction and desaturation (low oxygen levels).

An alternative to anaesthetist-delivered boluses of sedative drugs is the use of a pharmacokinetic infusion pump, as for example disclosed in US 8,038,645 B2. This type of approach can provide a smoother blood concentration of the sedative but is still delivered by the anaesthetist and therefore open to over and under sedation of the patient as described above.

Another option is patient-controlled sedation, in which the patient is at least partly in control of the amount of sedative they receive. The patient may control the sedation they receive using a switch, such as a push button, that is actuated to signal to a dose metering pump that more sedative is required. The dose metering pump then responds to the activation of the switch, within pre-set limits, which may for example limit the maximum amount capable of being administered over time. Repeated presses by the patient will cause gradual increases in the amount of sedative being administered until the patient considers the amount effective, or until a pre-set upper limit is reached. An advantage of this approach is that the patient is able to control their own sedation and thereby receive the optimum amount of sedative. A further advantage is that the total amount of sedative consumed has been shown in studies to be substantially reduced as compared with anaesthetist-administered sedation. In addition, patients able to control their own level of sedation tend to feel less anxious and have a shorter recovery time. To date there has been no commercially produced device capable of delivering patient controlled sedation using a pharmacokinetic pump to deliver the drug.

WO 03/057296 A1 discloses an apparatus and method for alleviating pain or discomfort while enabling safe drug delivery in correlation with monitoring of patient health conditions, in which a blood pressure cuff capable of being wrapped around a patient's wrist is affixed to a palm support portion with a depressible query response switch capable of being depressed by the patient's thumb and a drug dosage request switch is integrated into a finger support portion.

EP 1338295 A1 discloses a system for remotely controlling medication infusion and analyte monitoring, in which a monitoring device has a hosing and strap which allows it to be worn on a limbic region such as the arm, the device having a display and control keys for receiving input data and commands from the user.

US 2008/0220092 A1 discloses an automated programmable transdermal administrative system for providing pulsed doses of medications.

WO 2016/014311 A1 discloses a dose request device for a medicament delivery device including a housing, a dose request button, a short-range non-contact identifier, a communication interface configured to communicate with the medicament delivery device and a controller.

WO 2013/136176 A1 discloses an injection device having a miniaturized drug delivery portion, the device including a housing having an interior volume and the drug delivery portion having a volume enclosed within the housing.

EP 0523456 A1 discloses an apparatus and system for the self-dosing of a liquid machine, in which a push button is attached to a piston capable of being pressed by the patient a desired number of times corresponding to an amount of dosed liquid medicine.

### Summary of the Invention

In accordance with a first aspect of the invention there is provided a hand-operated device for a patient-controlled sedation system, the device comprising:
an elongate arcuate portion arranged to fit around a wrist of a patient; and
a switch arranged to activate upon compression across a width of the elongate arcuate portion,
wherein the elongate arcuate portion comprises a hollow tubular element formed of an elastomeric material,
wherein the switch is configured to be actuated by an increase in air pressure within the hollow tubular element upon compression of the elongate actuate portion.

An advantage is that fitting the device around the wrist of a patient and operating the switch by compression allows the device to be operated more easily by the patient. The device is also less likely to fall out of reach during an operation if it is attached around the patient's wrist.

To allow for the patient to identify when the switch has been operated, the device may have an indicator configured to provide one or more indications upon activation of the switch. The indicator may be one or more of: a light configured to illuminate the device upon activation of the switch; a vibration motor arranged to provide haptic feedback upon activation of the switch; and a sounder arranged to emit an audible signal upon activation of the switch.

The device may comprise a strap configured to connect proximal and distal ends of the elongate arcuate portion for securing the device around the patient's wrist.

The device may comprise a connection cable for connecting the switch to an interface for a dose metering device. In alternative examples the device may comprise a wireless transceiver for wirelessly transmitting an actuation signal when the switch is operated. The wireless transceiver may for example be a Bluetooth type transceiver.

The elastomeric material may for example be a silicone rubber. The hollow tubular element may be translucent or transparent, thereby enabling the element to be illuminated from within to provide visual feedback.

The switch may comprise a pressure sensor and a switching module, the switching module configured to receive a pressure signal from the pressure sensor and activate the switch when a sensed pressure exceeds a pre-set value.

In accordance with a second aspect there is provided a system for patient-controlled sedation, the system comprising:
a dose metering device arranged to provide a dose of a sedative to a patient; and
a hand-operated device according to the first aspect,
wherein the hand-operated device is connected to the dose metering device for controlling an amount of sedative provided to the patient upon operation of the switch.

### Detailed Description

The invention is described in further detail below by way of example and with reference to the accompanying drawings, in which:
figure 1 is a schematic diagram of an example system for patient-controlled sedation;
figure 2 is a drawing of an example patient interface for a patient-controlled sedation system;
figure 3 is a schematic sectional drawing of an example hand-operated device for a patient-controlled sedation system;
figure 4 is a drawing of an example hand-operated device for a patient-controlled sedation system;
figure 5 is a drawing of an alternative example hand-operated device for a patient-controlled sedation system; and
figure 6 is a schematic flowchart illustrating an example method of operating a patient-controlled sedation system.

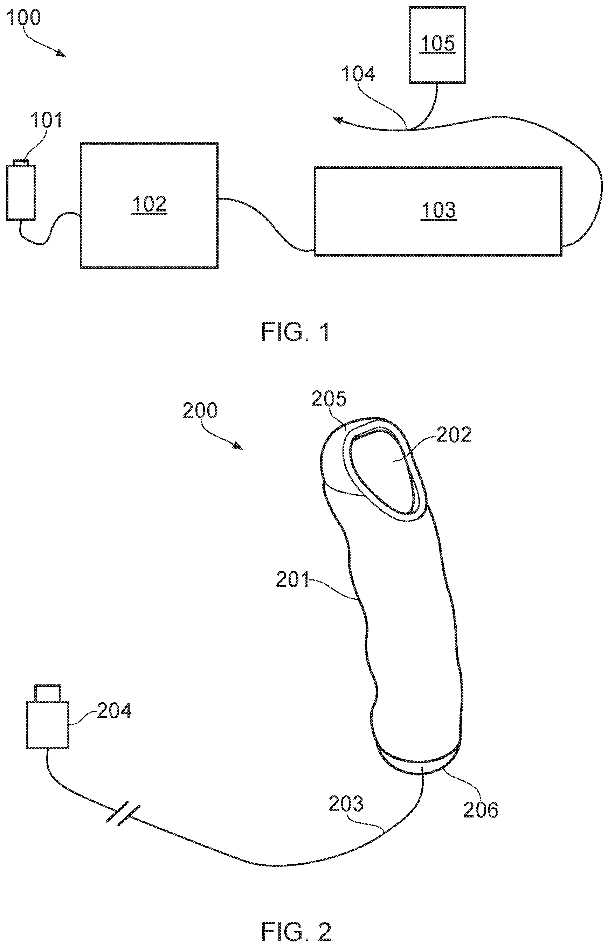

Figure 1 illustrates an example system 100 for patient-controlled sedation. The system 100 comprises a patient button interface 101 having a wired connection to a computer interface 102 for an anaesthetist. The computer interface 102 is connected to an infusion pump 103, which provides a controlled dose of sedative to a cannula 104 attached to a drip bag 105. The computer interface 102 may allow the anaesthetist to view the amount and rate of sedative being provided to the patient and to override this if necessary.

The anaesthetist-operated computer interface 102 may be a conventional (typically ruggedized) portable computer with a first two-way wired interface to the patient button interface 101 and a second two-way wired interface with the infuser pump 103. Both interfaces may for example be made using conventional USB connections. The infuser pump 103 may for example be a Perfusor fm^{®} infusion pump, available from B. Braun Melsungen AG. In some arrangements the computer interface 102 may be incorporated into the infusion pump 103.

An example hand-operated device 200 for use as a patient button interface 101 is illustrated in figure 2. The device 200 comprises a grip portion 201 for fitting within a patient's hand and a button 202 at a distal end 205 of the device 200. A cable 203 extends from a proximal end 206 of the device 200 for connecting the device 200 to a computer interface via a connector 204. In use, the patient grips the device 200 and presses the button 202 with their thumb to request an increase in the level of sedation.

A problem with the type of device 200 in figure 2 is that the patient may lose grip of the device 200 and not be able to operate the button 202. This may be partially solved by adding a wrist band to attach the device 200 to the patient's wrist. If the patient's grip loosens and the device falls but is held by the wrist band, the patient would need to pick up the device and reorient it before being able to press the button again. This may be difficult for the patient to do, particularly when partially sedated, and can potentially result in increased patient anxiety, which could lead to problems during an operation.

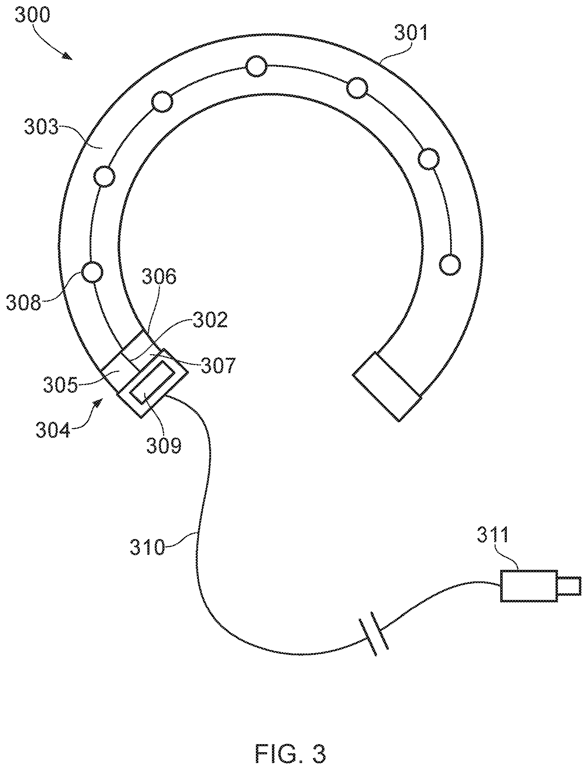

A schematic sectional view of an example hand-operated device 300 for a patient-controlled sedation system is shown in figure 3. The device 300 is in the form of a partial section of a ring, comprising an elongate arcuate portion 301 that is arranged to fit around the wrist of a patient. The inner diameter of the device 300 may therefore be in the region of around 5 to 10 cm. The device 300 comprises a switch 302 that is arranged to operate upon compression across a width of the elongate arcuate portion 301. The patient may operate the switch by squeezing the device 300 at any point along the length of the arcuate portion 301, making the operation significantly easier than that of finding and pressing a button, particularly when the patient is partially sedated. Being wrist-mounted, the device 300 is also far less prone to being misplaced and is always immediately to hand for the patient to operate.

A cable 310 connects a proximal end 304 of the device 300 to a connector 311 for connection to a dose metering device. Electrical power to the device 300 may be provided via the cable 310. In alternative examples the device 300 may comprise a wireless transceiver, for example a Bluetooth module, for wirelessly transmitting an actuation signal when the switch 302 is activated, and the device 300 may be powered internally with a battery. In a surgical operating environment, a cable connection may be preferable to avoid the possibility of interference with other sensitive electronic equipment.

The elongate arcuate portion 301 of the device 300 is formed of a flexible elastomeric material such as a silicone rubber, which allows the device to be positioned and held in place around the patient's wrist. The elongate arcuate portion 301 is hollow such that a compressive force applied at any point along the length of the portion 301 causes an increase in pressure within an internal volume 303, which is transmitted to the switch 302. The switch 302 then responds to the increase in pressure by activating, causing a signal to be sent to an infusion pump to request an increase in sedation. The switch 302 may for example be in the form of a pressure-activated switch that is configured to close above a pre-set pressure. Alternatively the switch 302 may comprise a pressure sensor 306 and a switching module 307 that receives a pressure signal from the pressure sensor 306 and activates the switch 302 when a sensed pressure exceeds a pre-set value. The switch 302 may be disposed at a proximal end 304 of the elongate arcuate portion 301. An advantage of using a pressure sensor 306 and switching module 307 is that the switch 302 may be activated not only according to the absolute value of pressure but also according to the length of time the pressure increase lasts. Spurious readings caused by momentary increases in pressure can thereby be discounted, and the switch 302 activated only when a sustained increase in pressure is applied. A further advantage is that the device 300 can be calibrated according to the particular patient before use, for example by having the patient apply pressure to the device 300 to set a pre-set value for subsequently activating the switch. An average pressure taken from a number of readings may be used. Different grip strengths can thereby be accommodated, for example allowing patients with reduced strength or impairment such as arthritis to use the device.

The device 300 may comprise a feedback mechanism to provide an indication to the patient of when the switch is activated. The feedback mechanism may involve haptic, aural or visual feedback. Haptic feedback may be provided by the device 300 comprising a vibratory motor 305, which is activated when the switch 302 is operated. The switching module 307 may be arranged to drive the vibratory motor 305 when activating the switch 302. Visual feedback may be provided by the device 300 comprising one or more lights 308 arranged to be lit when the switch 302 is activated. The lights 308 may be arranged in the internal volume 303 so that the device 300 is illuminated from within when the switch 302 is activated, which is particularly relevant when the arcuate portion 301 is in the form of a hollow tubular element composed of a translucent or transparent material such as a silicone rubber.

Aural feedback may be provided by the device 300 comprising a sounder 309 arranged to emit an audible alert such as a beep when the switch 302 is activated. The sounder may be activated by the switching module 307.

An advantage of the elongate arcuate portion 301 being in the form of a hollow tubular element formed of an elastomeric material, in combination with the switch 302 being activated by an increase in pressure being detected, is that the device may be used by the patient in different ways. A usual way of activating the switch 302 would be for the patient to wear the device 300 on their wrist and activate the switch 302 by squeezing with the other hand. The device 300 may alternatively be held around the patient's hand, where it will tend to stay in place by the resilient nature of the tubular element, and activated by squeezing the same hand. The device 300 may alternatively be used in other positions such as under the patient's chin, around the knee or in other positions where a squeezing action can be applied. Haptic feedback is particularly useful for when the device may be in positions that are not immediately visible to the patient and for environments that may have background noise that could make aural feedback indistinct.

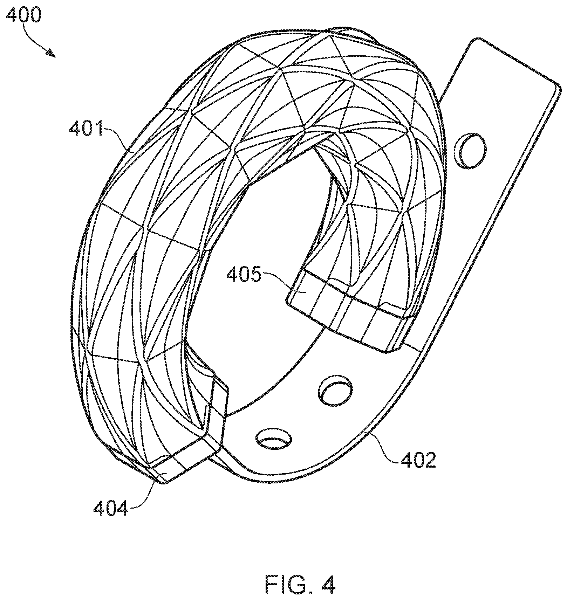

Figure 4 is a drawing of an example device 400 of the type shown in figure 3, in which the elongate arcuate portion 401 is a hollow ring formed of silicone rubber. An exterior surface of the elongate arcuate portion 401 is textured to improve grip and enable the patient to recognise the device by touch alone. A strap 402 removably connects the proximal and distal ends 404, 405 of the elongate arcuate portion 401 for securing the device 400 in place around a patient's wrist. The device 400 may comprise features described above in relation to the device 300 in figure 3.

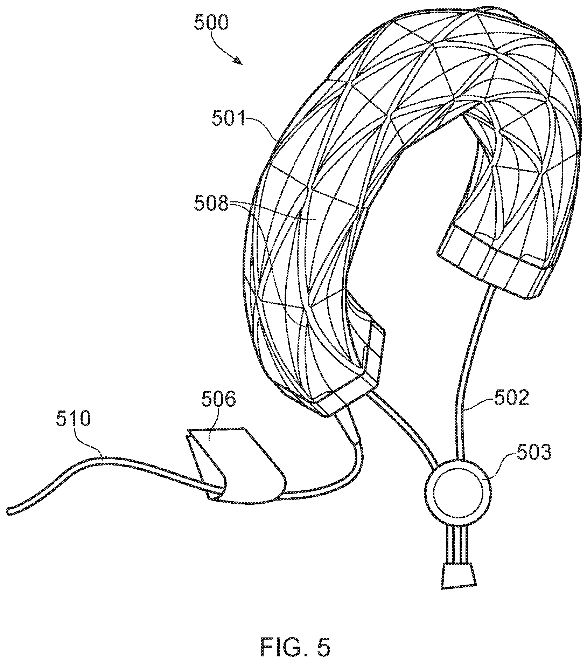

Figure 5 is a drawing of a further example device 500, in which an alternative way of securing the device 500 to the patient is used, in this case an adjustable strap 502 with a toggle 503 connects the proximal and distal ends 504, 505 of the elongate arcuate portion 501. A gown clip 506 may also be provided for securing the cable 510 to the patient's clothing. The device 500 comprises lights 508 within the hollow elongate arcuate portion 501 that illuminate the device 500 from within when the switch is activated. Other features described above in relation to the device 300 in figure 3 may also be present.

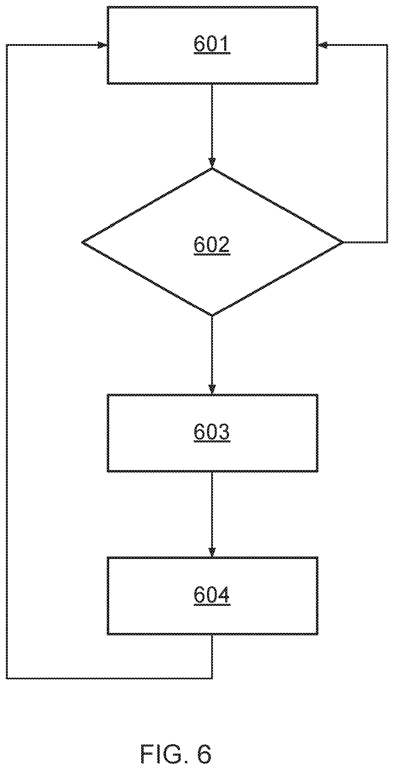

Figure 6 illustrates schematically an example method of operating a device of the type described herein, where the device comprises a pressure sensor and switching module together with a feedback mechanism. In a first step 601 the switching module obtains a pressure reading from the pressure sensor. In a second step 602, the switching module compares the pressure reading with a pre-set pressure threshold. If the pressure reading is below the pre-set pressure threshold, the method returns to step 601 and repeats. If the pressure reading is above the pre-set pressure threshold, at step 603 the switch is activated, sending a signal to request an increase in sedation level. At step 604 the switching module activates the feedback mechanism, for example a vibration motor, to provide feedback to the patient that the switch has been activated. The method then returns to step 601 and repeats. The method may pause before repeating the process to prevent multiple requests being transmitted from a single press. Step 602 may also involve determining whether the pressure threshold has been exceeded for a minimum time before proceeding to step 603 to prevent spurious momentary pressure increases from triggering an activation signal.

Other embodiments are intentionally within the scope of the invention as defined by the appended claims.

## Claims

1. A hand-operated device (300, 400) for a patient-controlled sedation system, the device (300, 400) comprising:
an elongate arcuate portion (301) arranged to fit around a wrist of a patient; and **characterized by**
a switch (302) arranged to activate upon compression across a width of the elongate arcuate portion (301),
wherein the elongate arcuate portion (301) comprises a hollow tubular element formed of an elastomeric material,
wherein the switch (302) is configured to be actuated by an increase in air pressure within the hollow tubular element upon compression of the elongate actuate portion (301).

2. The device (300) of claim 1 comprising an indicator (305, 308, 309) configured to provide one of more indications upon activation of the switch (302).

3. The device (300) of claim 2 wherein the indicator is one or more of:
a light (308) configured to illuminate the device upon activation of the switch (302);
a vibration motor (305) arranged to provide haptic feedback upon activation of the switch (302); and
a sounder (309) arranged to emit an audible signal upon activation of the switch (302).

4. The device (400) of any preceding claim comprising a strap (402) configure to connect proximal and distal ends (404, 405) of the elongate arcuate portion for securing the device (400) around a patient's wrist.

5. The device (300, 400) of any preceding claim comprising a connection cable (310) for connecting the switch (302) to an interface (102) for a dose metering device (103).

6. The device (300, 400) of claim 1 wherein the hollow tubular element is translucent or transparent.

7. The device (300, 400) of claim 1 wherein the switch (302) comprises a pressure sensor (306) and a switching module (307), the switching module (307) configured to receive a pressure signal from the pressure sensor (306) and activate the switch (302) when a sensed pressure exceeds a pre-set value.

8. A system for patient-controlled sedation (100), the system comprising:
a dose metering device (103) arranged to provide a dose of a sedative to a patient; and
a hand-operated device (300, 400) according to any preceding claim,
wherein the hand-operated device (300, 400) is connected to an interface (102) for controlling an amount of sedative provided to the patient by the dose metering device (103) upon operation of the switch (302).

## Patentansprüche

1. Handbetätigte Vorrichtung (300, 400) für ein patientengesteuertes Sedierungssystem, wobei die Vorrichtung (300, 400) umfasst:
einen länglichen, gebogenen Abschnitt (301), der so angeordnet ist, dass er um ein Handgelenk eines Patienten passt; und **gekennzeichnet durch**
einen Schalter (302), der so angeordnet ist, dass er beim Zusammendrücken über eine Breite des länglichen, gebogenen Abschnitts (301) aktiviert wird,
wobei der längliche, gebogene Abschnitt (301) ein hohles röhrenförmiges Element umfasst, das aus einem elastomeren Material gebildet ist,
wobei der Schalter (302) so konfiguriert ist, dass er durch einen Anstieg des Luftdrucks innerhalb des hohlen röhrenförmigen Elements beim Zusammendrücken des länglichen Betätigungsabschnitts (301) betätigt wird.

2. Vorrichtung (300) nach Anspruch 1, umfassend eine Anzeige (305, 308, 309), die so konfiguriert ist, dass sie bei Aktivierung des Schalters (302) eine oder mehrere Anzeigen bereitstellt.

3. Vorrichtung (300) nach Anspruch 2, wobei die Anzeige eines oder mehrere ist von:
einer Leuchte (308), die so konfiguriert ist, dass sie die Vorrichtung bei Aktivierung des Schalters (302) beleuchtet;
einem Vibrationsmotor (305), der so angeordnet ist, dass er bei Aktivierung des Schalters (302) eine haptische Rückmeldung bereitstellt; und
einen Tongeber (309), der so angeordnet ist, dass er bei Aktivierung des Schalters (302) ein hörbares Signal emittiert.

4. Vorrichtung (400) nach einem der vorstehenden Ansprüche, umfassend einen Riemen (402), der so konfiguriert ist, dass er das proximale und das distale Ende (404, 405) des länglichen, gebogenen Abschnitts verbindet, um die Vorrichtung (400) um das Handgelenk eines Patienten zu befestigen.

5. Vorrichtung (300, 400) nach einem vorstehenden Anspruch, umfassend ein Verbindungskabel (310) zum Verbinden des Schalters (302) mit einer Schnittstelle (102) für eine Dosismessvorrichtung (103).

6. Vorrichtung (300, 400) nach Anspruch 1, wobei das hohle röhrenförmige Element lichtdurchlässig oder transparent ist.

7. Vorrichtung (300, 400) nach Anspruch 1, wobei der Schalter (302) einen Drucksensor (306) und ein Schaltmodul (307) umfasst, wobei das Schaltmodul (307) so konfiguriert ist, dass es ein Drucksignal von dem Drucksensor (306) empfängt und den Schalter (302) aktiviert, wenn ein erfasster Druck einen voreingestellten Wert überschreitet.

8. System zur patientengesteuerten Sedierung (100), wobei das System umfasst:
eine Dosismessvorrichtung (103), die so angeordnet ist, dass sie einem Patienten eine Dosis eines Sedativums bereitstellt; und
eine handbetätigte Vorrichtung (300, 400) nach einem der vorstehenden Ansprüche,
wobei die handbetätigte Vorrichtung (300, 400) mit einer Schnittstelle (102) verbunden ist, um eine Menge eines Sedativums zu steuern, die dem Patienten durch die Dosismessvorrichtung (103) bei Betätigung des Schalters (302) bereitgestellt wird.

## Revendications

1. Dispositif (300, 400) à commande manuelle pour un système de sédation contrôlé par le patient, le dispositif (300, 400) comprenant :
une partie (301) arquée allongée agencée pour s'adapter autour d'un poignet d'un patient ; et **caractérisée en ce que**
un commutateur (302) agencé pour s'activer lors d'une compression sur une largeur de la partie (301) arquée allongée,
dans lequel la partie (301) allongée arquée comprend un élément tubulaire creux formé d'un matériau élastomère,
dans lequel le commutateur (302) est configuré pour être actionné par une augmentation de la pression d'air à l'intérieur de l'élément tubulaire creux lors de la compression de la partie (301) arquée allongée.

2. Dispositif (300) selon la revendication 1 comprenant un indicateur (305, 308, 309) configuré pour fournir l'une des indications supplémentaires lors de l'activation du commutateur (302).

3. Dispositif (300) selon la revendication 2 dans lequel l'indicateur est un ou plusieurs parmi :
une lumière (308) configurée pour éclairer le dispositif lors de l'activation du commutateur (302) ;
un moteur (305) de vibration agencé pour fournir un retour haptique lors de l'activation du commutateur (302) ; et
une sirène (309) agencée pour émettre un signal sonore lors de l'activation du commutateur (302).

4. Dispositif (400) selon une quelconque revendication précédente comprenant une sangle (402) configurée pour connecter les extrémités (404, 405) proximale et distale de la partie arquée allongée pour fixer le dispositif (400) autour du poignet d'un patient.

5. Dispositif (300, 400) selon une quelconque revendication précédente comprenant un câble (310) de connexion pour connecter le commutateur (302) à une interface (102) pour un dispositif (103) de dosage.

6. Dispositif (300, 400) selon la revendication 1 dans lequel l'élément tubulaire creux est translucide ou transparent.

7. Dispositif (300, 400) selon la revendication 1 dans lequel le commutateur (302) comprend un capteur (306) de pression et un module (307) de commutation, le module (307) de commutation étant configuré pour recevoir un signal de pression du capteur (306) de pression et activer le commutateur (302) lorsqu'une pression détectée dépasse une valeur préréglée.

8. Système (100) de sédation contrôlée par le patient, le système comprenant :
un dispositif (103) de dosage agencé pour fournir une dose d'un sédatif à un patient ; et
un dispositif (300, 400) à commande manuelle selon une quelconque revendication précédente,
dans lequel le dispositif (300, 400) à commande manuelle est connecté à une interface (102) pour contrôler une quantité de sédatif fournie au patient par le dispositif (103) de dosage lors de l'actionnement du commutateur (302).
